# EUROPEAN PATENT SPECIFICATION

(11) **EP 4 616 895 B1**
(45) Date of publication and mention of the grant of the patent: **29.07.2026**
(21) Application number: 24222511.8
(22) Date of filing: 20.12.2024
(51) Int. Cl.: A61M 25/01

(54) **ADJUSTABLE TENSIONING SPOOL FOR STEERABLE CATHETERS**
EINSTELLBARE SPANNSPULE FÜR LENKBARE KATHETER
BOBINE DE TENSION RÉGLABLE POUR CATHÉTERS ORIENTABLES

(30) Priority: 11.03.2024 US 202463563562 P
(43) Date of publication of application: 17.09.2025
(73) Proprietor: Cephea Valve Technologies, Inc., Abbott Park, Illinois 60064 (US)
(72) Inventor: Valencia, Francisco, East Palo Alto, 94303 (US); Basilio, Elimar, Sunnyvale, 94089 (US); Monteon, Gabriel, Hollister, 95023 (US)
(74) Representative: LKGLOBAL Partnerschaftsgesellschaft mbB

(56) References cited:
- US-A1- 2007 118 155
- US-A1- 2016 287 840

## Description

### Background of the Disclosure

The present disclosure relates generally to devices, systems and methods (not claimed) for delivering an interventional device into a patient for implantation.

Intravascular medical procedures allow for the performance of therapeutic treatments in a variety of locations within a patient's body while requiring only relatively small access incisions. An intravascular procedure may, for example, eliminate the need for open-heart surgery, reducing risks, costs, and time associated with an open-heart procedure. The intravascular procedure may also enable faster recovery times with lower associated costs and risks of complications. An example of an intravascular procedure that reduces procedure and recovery time and cost over conventional open heart surgery is a heart valve replacement or repair procedure in which an artificial valve or valve repair device is guided to the heart through the patient's vasculature. For example, a catheter is inserted into the patient's vasculature and directed to the inferior vena cava. The catheter is then urged through the inferior vena cava toward the heart by applying force longitudinally (*e.g.,* pushed forward) to the catheter. Upon entering the heart from the inferior vena cava, the catheter enters the right atrium. The catheter may be guided across the atrial septum (*e.g.,* via a guidewire that has already been passed through the atrial septum) into the left atrium. The distal end of the catheter may be deflected by one or more deflecting mechanisms in order to align the distal end of the catheter, as well as a medical device positioned therein, with the mitral valve. Catheter deflection can be achieved by tension cables, or other mechanisms positioned inside the catheter. Precise control of the distal end of the catheter allows for more reliable and faster positioning of a medical device and/or implant and other improvements in the procedures.

US 2026/0287840 A1 describes methods and apparatuses for detecting tension on a tendon and/or mechanical deformation (e.g., breakage) of one or more steering tendon of a steerable and flexible articulating device. Theses apparatuses may have one or more tendons that are each electrically conductive and configured to steer the apparatus when tension is applied to the proximal end of the tendon. Tension and/or breakage (or other deformation) of one or more of these tendons may be detected by monitoring the electrical resistance of the tendons.

### Brief Summary

The aforementioned objectives are achieved according to the invention by means of a catheter system according to claim 1. Preferred embodiments are defined in the dependent claims.

According to one aspect of the disclosure, a catheter system includes a steering catheter having a wall and a first lumen formed within the wall. A first steering cable may extend through the first lumen along a length of the wall of the steering catheter toward a distal end portion of the steering catheter, the first steering cable being coupled to the distal end portion of the steering catheter. A steering assembly may include a first spool assembly, the first spool assembly including a first outer spool, a first inner spool received at least partially within a recess of the first outer spool, and a spool shaft having a first end portion received at least partially within a recess of the first inner spool. The first end portion of the spool shaft and the recess of the first inner spool may each be shaped so that rotation of the spool shaft about a longitudinal axis thereof transmits torque to the first inner spool to cause rotation of the first inner spool about the longitudinal axis of the spool shaft. The first inner spool may include a spline section forming a plurality of splines, and the recess of the first outer spool may have a shape that is complementary to the plurality of splines, such that when the first inner spool is received at least partially within the recess of the first outer spool, the plurality of splines engage the complementary shape of the recess of the first outer spool to prevent rotation of the first inner spool relative to the first outer spool about the longitudinal axis of the spool shaft. The first steering cable may be fixed to the first outer spool and configured to be wound around the first spool assembly upon rotation of the first outer spool about the longitudinal axis. The first inner spool may be configured to be received within the first outer spool in any one of a plurality of unique relative rotational positions, so that the first steering cable is configured to have a first amount of pretension in a first one of the plurality of unique relative rotational positions and a second amount of pretension in a second one of the plurality of unique relative rotational positions, the first amount of pretension being different than the second amount of pretension. The steering assembly may include a base, the spool shaft may have a central portion received within the base, and the first spool assembly may be positioned on a first surface of the base. The steering assembly may include a second spool assembly positioned on a second surface of the base opposite the first surface of the base. The second spool assembly may include a second outer spool, and a second inner spool received at least partially within a recess of the second outer spool. The first inner spool may be identical to the second inner spool, and the first outer spool may be identical to the second outer spool. The spool shaft may have a second end portion received at least partially within a recess of the second inner spool, and the second end portion of the spool shaft and the recess of the second inner spool may each be shaped so that rotation of the spool shaft about the longitudinal axis thereof transmits torque to the second inner spool to cause rotation of the second inner spool about the longitudinal axis of the spool shaft. The second inner spool may include a spline section forming a plurality of splines, and the recess of the second outer spool may have a shape that is complementary to the plurality of splines of the spline section of the second inner spool, such that when the second inner spool is received at least partially within the recess of the second outer spool, the plurality of splines of the spline section of the second inner spool engage the complementary shape of the recess of the second outer spool to prevent rotation of the second inner spool relative to the second outer spool about the longitudinal axis of the spool shaft.

The catheter system may further include a second lumen formed within the wall of the steering catheter, a second steering cable extending through the second lumen along a length of the wall of the steering catheter toward the distal end portion of the steering catheter, the second steering cable being coupled to the distal end portion of the steering catheter. The second steering cable may be fixed to the second outer spool and configured to be wound around the second spool assembly upon rotation of the second outer spool about the longitudinal axis. The first steering cable may be fixed to the first outer spool and the second steering cable may be fixed to the second outer spool such that, when the spool shaft is rotated about the longitudinal axis to cause simultaneous rotation of the first outer spool and the second outer spool, the first steering cable winds around the first spool assembly while the second steering cable unwinds from the second spool assembly. The first lumen may be positioned on a generally opposite side of the steering catheter compared to the second lumen. Tensioning the first steering cable may be configured to deflect the distal end portion of the steering catheter in a first steering direction, and tensioning the second steering cable may be configured to deflect the distal end portion of the steering catheter in a second steering direction, the first steering direction being generally opposite the second steering direction. The first lumen may be positioned diametrically opposite to the second lumen. Tensioning the first steering cable may be configured to deflect the distal end portion of the steering catheter in a first steering direction, and tensioning the second steering cable may be configured to deflect the distal end portion of the steering catheter in a second steering direction, the first steering direction and the second steering direction being positioned in a same plane but in opposite directions along the same plane. The system may further include a drive gear, the drive gear having a central opening, the second end portion of the spool shaft being received within the central opening. The second end portion of the spool shaft and the central opening recess of the drive gear may each be shaped so that rotation of the drive gear about the longitudinal axis of the spool shaft transmits torque to the spool shaft to cause rotation of the spool shaft about the longitudinal axis of the spool shaft.

The steering assembly may further include an actuator shaft having a first end portion and a second end portion, the second end portion being received within and coupled to the base. The steering assembly may further include a gear wheel having a central opening, the first end portion of the actuator shaft being received within the central opening of the gear wheel such that rotation of the actuator shaft about a longitudinal axis thereof causes rotation of the gear wheel about the longitudinal axis of the actuator shaft. The gear wheel may be operably coupled to the drive gear such that rotation of the gear wheel transmits torque to the drive gear. The steering assembly may be received within a handle of a delivery system, and the actuator shaft may have a terminal end protruding outside of the handle. A steering knob may be coupled to the terminal end of the actuator shaft such that rotation of the steering knob causes corresponding rotation of the actuator shaft. The steering assembly may include a first hard stop operably coupled to the drive gear, the first hard stop configured to prevent rotation of the drive gear beyond a first threshold amount of rotation in a first rotational direction. The steering assembly may include a second hard stop operably coupled to the drive gear, the second hard stop configured to prevent rotation of the drive gear beyond a second threshold amount of rotation in a second rotational direction opposite the first rotational direction.

### Brief Description of the Drawings

Fig. 1 is a perspective view of a delivery system configured for delivering, positioning, and deploying a prosthetic heart valve, including a handle assembly and a catheter assembly.
Fig. 2 is an exploded view of a portion of the handle assembly of the delivery system of Fig. 1, including valve cover retraction knob and cone assembly.
Fig. 3 is a side view of a portion of the catheter assembly of the delivery system of Fig. 1, including a catheter shaft, coiled and/or braided section, valve cover, and nosecone.
Fig. 4A is a transverse cross-sectional view of the catheter shaft of Fig. 3, showing various components thereof.
Figs. 4B-4C are transverse cross-sectional views of a catheter assembly, operable with the delivery system of Fig. 1, according to another aspect of the disclosure.
Fig. 5A is a longitudinal cross-section of the catheter assembly of Fig. 3, showing the nested arrangement of various components thereof.
Fig. 5B is a longitudinal cross-section of a catheter assembly, operable with the delivery system of Fig. 1 and according to another aspect of the disclosure, showing the nested arrangement of various components thereof.
Fig. 6 is an isolated view of a suture rigging assembly that may be coupled to a suture catheter of any of the delivery systems described herein.
Fig. 7 is a perspective view of the suture rigging assembly connecting the suture catheter to the prosthetic heart valve.
Figs. 8-9 are top perspective views of portions of a steering assembly according to an aspect of the disclosure.
Fig. 10 is a cross-section of the portion of the steering assembly of Figs. 8-9.
Fig. 11 is a perspective view of a spool shaft of the steering assembly of Figs. 8-10.
Fig. 12 is a perspective view of an outer spool of the steering assembly of Figs. 8-10, shown in partial phantom.
Fig. 13 is a perspective view of an inner spool of the steering assembly of Figs. 8-10.

### Detailed Description

As used herein, the term "inflow end," when used in connection with a prosthetic heart valve, refers to the end of the heart valve through which blood enters when the heart valve is functioning as intended, whereas the term "outflow end," when used in connection with a prosthetic heart valve, refers to the end of the heart valve through which blood exits when the heart valve is functioning as intended. For a prosthetic mitral valve, the inflow end is closest to the left atrium when the heart valve is implanted in a patient, and the outflow end is closest to the left ventricle when the heart valve is implanted in a patient. Further, when used herein in connection with a delivery device, the terms "proximal" and "distal" are to be taken as relative to a user operating the device in an intended manner. "Proximal" is to be understood as relatively close to the user and "distal" is to be understood as relatively farther away from the user. Also as used herein, the terms "substantially," "generally," and "about" are intended to mean that slight deviations from absolute are included within the scope of the term so modified.

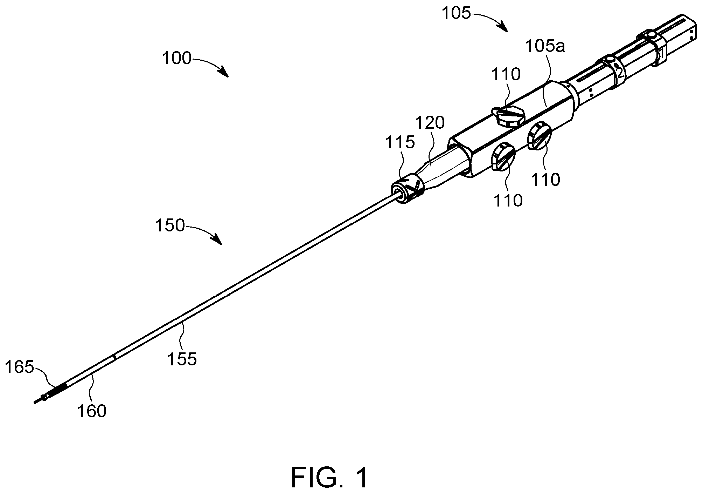

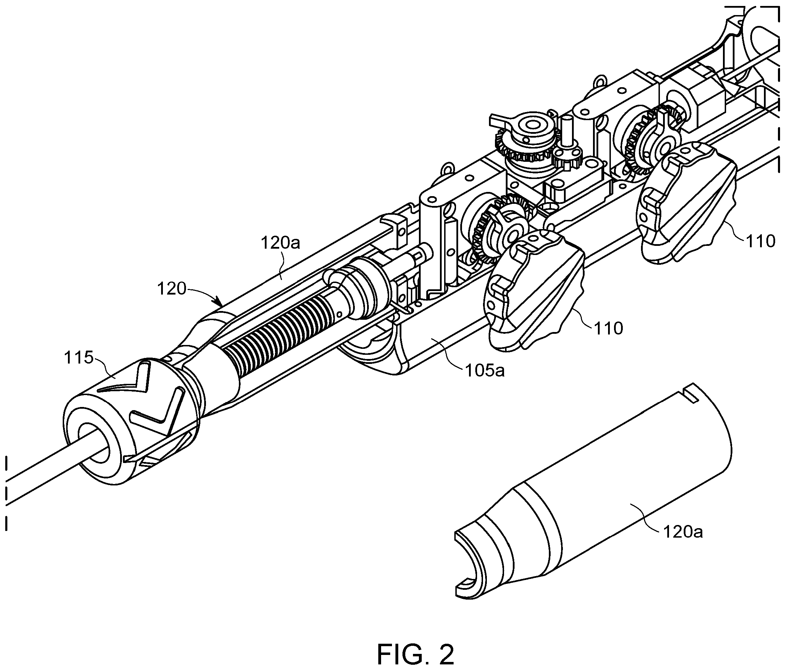

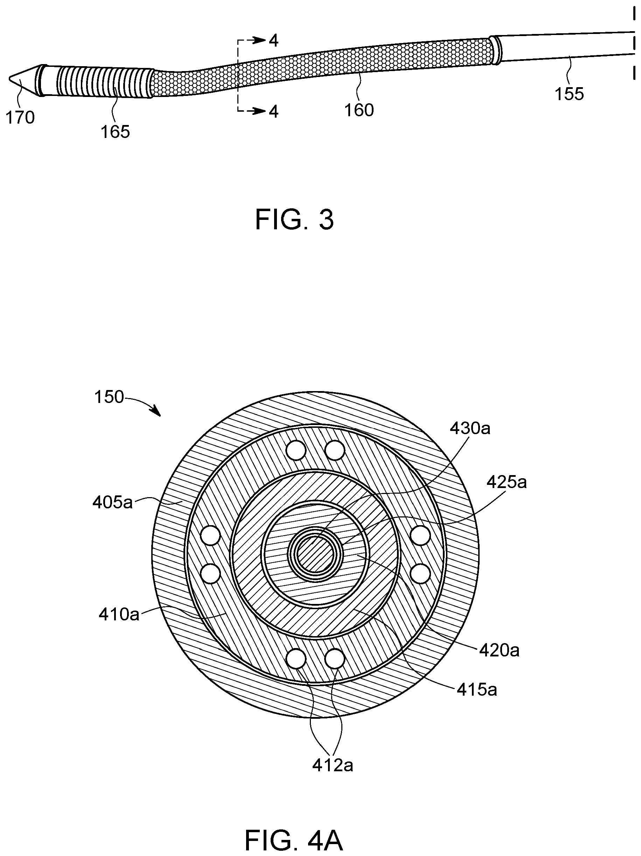

Fig. 1 is a perspective view of an example of a delivery system 100 configured for delivering, positioning and deploying a prosthetic heart valve, including a handle assembly 105 and a catheter assembly 150. Fig. 2 is an exploded view of a portion of the handle assembly 105 of the delivery system 100 of Fig. 1, including valve cover retraction knob 115 and cone assembly 120 (which may also be referred to as a locking assembly herein). Fig. 3 is a side view of a distal end portion of the catheter assembly 150 of the delivery system 100 of Fig. 1, including a catheter shaft 155, coiled and/or braided section 160, valve cover 165 (which may also be referred to as a valve capsule), and an atraumatic distal tip or nosecone 170.

Delivery system 100 generally includes a handle assembly 105 and a catheter assembly 150. Catheter assembly 150 extends from a proximal end coupled to handle assembly 105 to an atraumatic tip or nosecone 170 (not shown in Fig. 1) at a distal end and includes a plurality of catheter and/or hypotube components, at least some of which are longitudinally slidable relative to one another and provide different functionality during operation of delivery system 100 to enable effective delivery and deployment of a prosthetic heart valve, such as a prosthetic mitral valve. While the catheter assembly 150 is depicted as having a length relative to handle assembly 105, it should be understood that the catheter assembly 150 can have any suitable length and, in some examples, has a much greater length than the handle assembly 105.

Attached to a housing 105a of the handle assembly 105 can be various control mechanisms for controlling one or more aspects of the delivery. For example, attached to the housing 105a of the handle assembly 105 can be one or more knobs 110 for controlling steering of the of the catheter assembly 150. Additional details regarding steering operations that may be utilized in conjunction with the components and features described herein are described in U.S. Patent Application Publication No. 2023/0364387. The handle assembly 105 can also include a valve cover retraction knob 115 and a locking or cone assembly 120. The valve cover retraction knob 115 can be rotated to allow for retraction of valve cover 165 during delivery and/or expression of a prosthetic heart valve. In some examples, rotating valve cover retraction knob 115 results in proximal translation of a catheter member that is attached to the valve cover 165, allowing for the valve cover 165 to uncover a self-expandable prosthetic heart valve to allow the prosthetic heart valve to self-expand. The cone assembly 120 can be rotated relative to housing 105a to lock or unlock an outer catheter relative to an inner catheter, although the functionality of the cone assembly 120 is not described in greater detail herein. In some embodiments, the cone assembly 120 may be omitted.

The catheter assembly 150 can include a catheter shaft 155, a coiled and/or braided section 160, a valve cover 165, and a nosecone 170 (which is not shown in Fig. 1), as will be described in greater detail below. It should also be understood that catheter assembly 150 may include a number of additional catheter members nested within catheter shaft 155, as described in greater detail below.

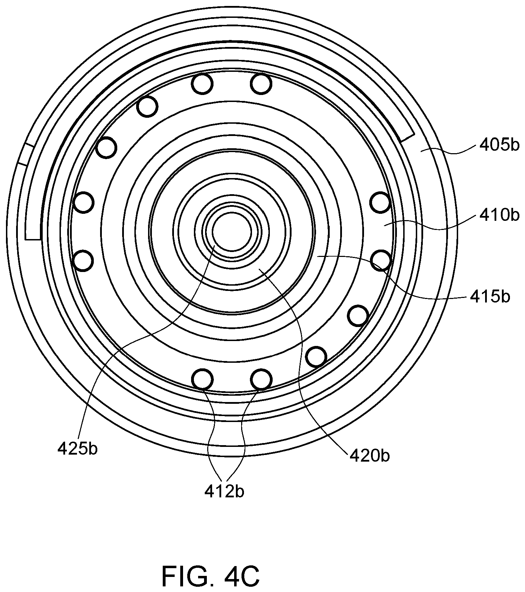

Fig. 4A is a transverse cross-sectional view of the catheter shaft 155 of Fig. 3, showing various components thereof, including addition catheter components nested therein.

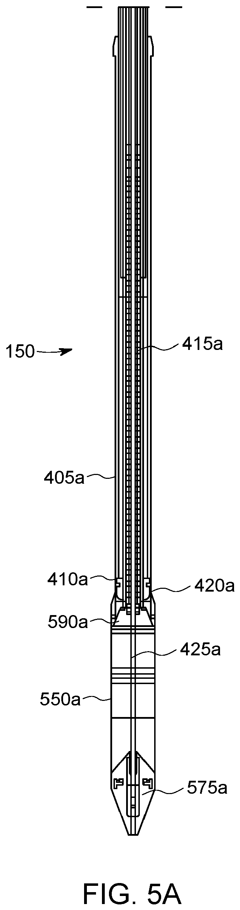

As shown in Fig. 4A, which is a transverse cross-sectional view of catheter assembly 150 taken along line 4-4 of Fig. 3, these components may include an outer sheath 405a (which may include the catheter shaft 155, coiled and/or braided section 160, and/or valve cover 165), a steering catheter 410a, an extension catheter 415a, a suture catheter 420a, and a nosecone catheter 425a, all arranged in a concentric nested relationship. The arrangement of these components, as well as valve cover 550a, nosecone 575a, and valve retainer 590a (also referred to as a "can") is shown in the longitudinal cross-section of catheter assembly 150 shown in Fig. 5A. During delivery, the proximal petals (*e.g.* the proximal apices of a frame, such as an outer frame) of the compressed prosthetic valve can be nested inside of the valve retainer 590 when the prosthetic valve is held back by the suture catheter, which is under tension. In one example, valve retainer 590a may be attached directly to a tip ring of the steering catheter 410a. As illustrated in the figures, nosecone catheter 425a has a lumen sized to receive a guidewire 430a therein. Each of these components is described in detail below. It should be understood that the valve cover 165 of Fig. 1 and Fig. 3 may be substantially the same as or identical to the other valve covers described herein. Similarly, it should be understood that the atraumatic distal tip or nosecone 170 of Fig. 3 may be substantially the same as or identical to the other atraumatic distal tips or nosecones described herein. In some examples, the suture catheter 420a may have a plurality of sutures or other wire loops attached to a distal end thereof, the suture loops or wire loops configured to selectively maintain a connection between the prosthetic heart valve and the delivery device.

To selectively control the curvature of a distal section of steering catheter 410a, the steering catheter 410a may be provided with a plurality of tension cables (not shown). The tension cables may travel from handle assembly 105 (*e.g.,* one or more of the knobs 110) through a plurality of lumens 412a (which in some embodiments may be polymer tubes, such as Nylon, Pebax, polyimide, or polytetrafluoroethylene or "PTFE" tubes, or any other type of polymer) to the distal end of steering catheter 410a. In one embodiment, steering catheter 410a may include four lumens 412a equally spaced at 90° intervals around the circumference of steering catheter 410a. In the embodiment of Fig. 4A, steering catheter 410a may include four pairs of lumens 412a (a total of eight lumens 412a), with the pairs of lumens equally spaced at 90° intervals around the circumference of steering catheter 410a. Any number of these lumens 412a (or pairs of lumens) may be provided depending on the various directions of deflection that may be desired. For example, six pairs of lumens 412a may be provided, with each pair of lumens 412a housing one steering cable of a pair of steering cables coupled to a corresponding steering knob 110 (*e.g.,* two steering cables attached to each steering knob 110, with each steering cable extending through one pair of lumens 412a, for a total of twelve lumens 412a arranged in six pairs).

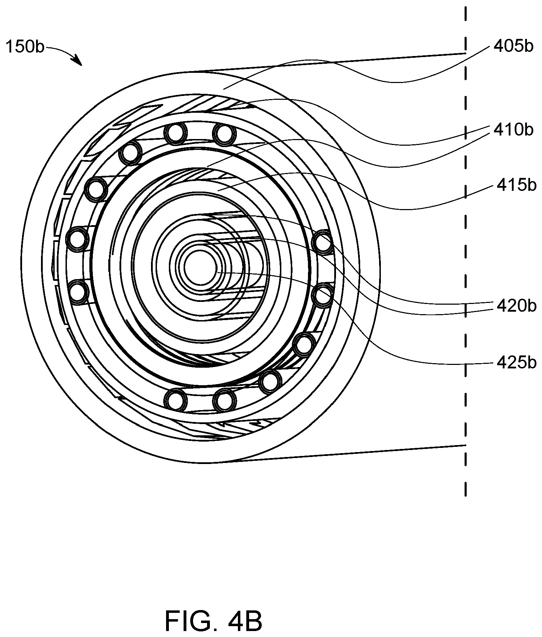

Figs. 4B-4C are transverse cross-sectional views of a catheter assembly 150b, operable with the delivery system of Fig. 1 (*e.g.,* in place of catheter assembly 150), according to another aspect of the disclosure.

In this example, catheter assembly 150b omits an extension catheter (*e.g.,* extension catheter 415a described above) and includes an outer catheter 405b (which may include the catheter shaft, coiled and/or braided section, and/or valve cover similar to those shown and described in connection with Fig. 3), a steering catheter 410b, an intermediate catheter 415b, a suture catheter 420b, and a nosecone catheter 425b. Also in this example, the steering catheter 410b includes six pairs (a total of 12) of lumens 412b, which again may be formed with polymer tubes.

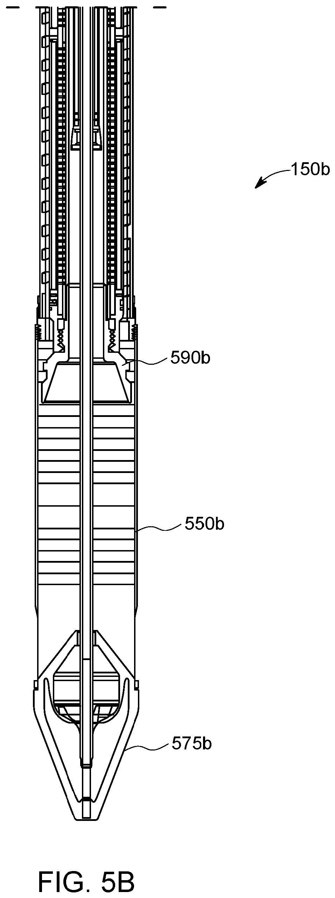

The arrangement of these components, as well as valve cover 550b, nosecone 575b, and valve retainer 590b is shown in the longitudinal cross-section of catheter assembly 150b shown in Fig. 5B.

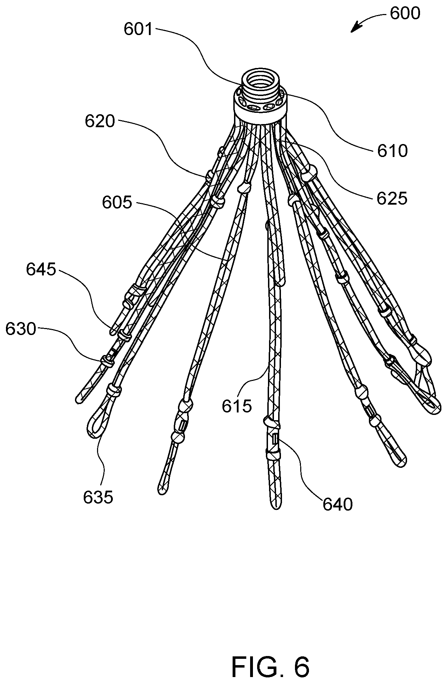

A suture rigging assembly 600 that assists in collapsing and drawing prosthetic heart valve V into delivery device 100 is shown in Figs. 6-7. Suture rigging assembly 600 may also help keep the prosthetic heart valve V connected to the delivery device 100, as well as helping control the deployment of the prosthetic heart valve V during use. Suture rigging assembly 600 may include a coupling ring 601 to which a plurality of suture tethers may be attached. An exemplary suture rigging assembly and coupling ring is shown in U.S. Pre-Grant Publication 2023/0030110.

One or more suture threads 605 may be attached to the head of coupling ring 601. The suture threads 605 may be comprised of various materials, both man-made and natural. Examples of natural suture materials may include, but are not limited to, silk, linen, and catgut. Examples of synthetic suture materials may include, but are not limited to, textiles such as nylon or polyester, or flexible metallic cables. An elongated suture thread 605 may be threaded through a plurality of the bores 610 in coupling ring 601 to form tethers 615. Suture rigging assembly 600 may include a coupling ring 601 having at least one tether 615 or a plurality of tethers. For example, suture thread 605 may be threaded distally through a bore 610 in inner ring of coupling ring 601 and then proximally though an adjacent bore in outer ring of coupling ring 601, thereby forming an elongated loop or tether 615 extending distally from coupling ring 601. Thus, tether 605 includes two lengths of suture thread extending side-by-side and continuous with one another at their distal ends. Suture thread 605 may then be threaded distally through an adjacent bore 610 in inner ring and then proximally through an adjacent bore in outer ring, thereby forming another elongated loop or tether 615 extending distally from coupling ring 601. This pattern may be repeated to form a plurality of elongated loops or tethers 615 around the entire circumference of coupling ring 601. Thus, tethers 615 may be formed by a single continuous suture thread 605, with the leading and trailing ends of the suture thread being joined to one another by one or more terminating knots.

Suture threads forming a tether 615 may be joined together by a first knot or stop knot 620 at a spaced distance from coupling ring 601. Stop knots 620 reduce the ability of suture threads to separate too far from one another or to create a large loop or lasso. The distance between the knots on a tether 615 will define the maximum loop or lasso that can be formed by the tether. As a result of using knots, any loop or lasso able to form will be smaller in size than the loop or lasso in a tether 615 that does not have any knots. Preventing the formation of large loops or lassos is important because a large loop or lasso may become entangled with the apexes of the ventricular anchor of a prosthetic heart valve, thereby impairing the user's ability to pull back the entangled tether 615 after valve has been deployed. As shown in Fig. 6, stop knots 620 in adjacent tethers 615 preferably are formed at different spaced distances from coupling ring 601. For example, the stop knots 620 in a first group of tethers 615 may be spaced a first distance from coupling ring 601, and the stop knots in a second group of tethers that alternate with the tethers in the first group may be spaced a second distance greater than the first distance from the coupling ring. By offsetting the stop knots 620 in adjacent tethers 615 from one another, the tethers are better able to collapse to a smaller, more compact cross-sectional size within the confines of delivery device 100.

The stop knots 620 in tethers 615 create in each tether an upper or proximal connecting loop 625 between the knot and coupling ring 601. The ability of suture thread 605 to move freely within bores 610 enables the lengths of tethers 615 to self-adjust to a certain degree. That is, each tether 615 is free to move proximally until its stop knot 620 contacts coupling ring 601 and is free to move distally until the stop knots in the adjacent tethers contact the coupling ring. Therefore, as one tether 615 lengthens as it moves distally, there is a corresponding proximal movement and shortening of the adjacent tethers on either side of it, in the manner of a pulley. This adjustment in the lengths of tethers 615 enables a balancing of the load imparted to each of the tethers as prosthetic heart valve is collapsed during loading into delivery device 100 or during re-sheathing. For example, if a shorter tether 615 experiences a higher tensile stress upon the loading of prosthetic heart valve into delivery device 100, that tether may lengthen as the adjacent tethers shorten until the tensile stress on all of the tethers reaches an equilibrium point at which the total tensile stress is substantially evenly distributed among all of the tethers. Maintaining a balanced load among tethers 615 prevents any one of the tethers from becoming overloaded and breaking, which can impede the functionality of the entire system. Further, more evenly distributing the load among tethers 615 enables the overall tensile capacity of suture rigging assembly 600 to be increased.

Additional knots may also be formed at the distal or closed end of tethers 615. As shown in Fig. 6, a third knot or lower fixture knot 630 may be formed at a spaced distance from the distal end of tether 615, forming a closed attachment loop 635 therein. Attachment loops 635 are intended to hook onto the pins of prosthetic heart valve and to apply tension to assist in collapsing the prosthetic heart valve during loading into delivery device 100, as described more fully below. Preferably, attachment loops 635 have a relatively small size so that, following their release from pins during deployment, they do not become entangled with the pins or other structures of prosthetic heart valve, impeding proper deployment of the heart valve and removal of delivery device 100 from the patient.

To help visualize the locations of tethers 615, and in particular the positions of attachment loops 635, during the deployment of prosthetic heart valve in a patient, some embodiments of suture rigging assembly 600 may include a radiopaque marker 640 on all or at least some of the tethers. Radiopaque markers 640 may be formed of any material that can be readily visualized under fluoroscopy, including metals such as gold, platinum, platinum-iridium, tantalum, tantalum-tungsten, and others, and may take any shape. Preferably, radiopaque markers 640 have a bore or channel extending therethrough so that the markers may be threaded onto suture threads before lower fixture knot 630 is formed therein or as suture thread 605 is threaded through bores 610. In some embodiments, radiopaque markers 640 may be cylindrical, with a bore extending therethrough along the longitudinal axis of the cylinder. The radiopaque markers 640 provided on suture rigging assembly 600 need not all have the same shape, and different shapes may be assembled to various tethers 615 to indicate the orientation of prosthetic heart valve or to identify various portions thereof. Moreover, if any of tethers 615 is improperly affixed to prosthetic heart valve or becomes improperly affixed to the prosthetic heart valve during delivery of the heart valve into the patient or during deployment, radiopaque markers 640 may help to identify which of the tethers is improperly affixed and identify its location.

Radiopaque markers 640 may be held in a fixed position on tethers 615 by lower fixture knot 630 at the distal end of the marker and by a second or upper fixture knot 645 formed in the tether at the proximal end of the marker. Fixture knots 645 and 630 capture the radiopaque marker 640 therebetween and prevent it from sliding along the length of tether 615 toward or away from attachment loop 635. As a less preferable alternative, adhesives can be used to attach the radiopaque markers 640 at fixed positions to tethers 615. As a result, once a radiopaque marker 640 has been identified under fluoroscopy, the user will know the position of the attachment loop 635 associated with that marker.

The use of knots to form suture rigging assembly 600 provides several advantages. Firstly, it enables adhesives to be avoided, reducing sterilization, storage and biocompatibility issues that adhesives may create. The elimination of adhesives may also reduce the formation of very small particles during the use of delivery device 100, which particles could potentially be released into the patient's bloodstream. The use of knots throughout suture rigging assembly 600 also enables the assembly to be self-balancing, minimizing the tensile stress in any one tether 615 and increasing the overall tensile capacity of the suture rigging assembly. Finally, the various knots in each tether 615 keeps suture threads close to one another to prevent undesirable entanglement of the tethers with structures of prosthetic heart valve during deployment.

Suture rigging assembly 600 can be used to attach, load, and release a wide variety of heart valves to/from a wide variety of catheter-based delivery systems. Thus, suture rigging assembly 600 is designed to attach to a prosthetic heart valve and sustain a tensile load path between the heart valve and a delivery device as the heart valve is retracted into a sheath of the delivery device.

One way in which suture rigging assembly 600 may be used to collapse and load prosthetic heart valve V into the valve cover (*e.g.,* valve cover 165, 550a, or 550b) of delivery device 100 will now be described. Initially, suture rigging assembly 600 is attached to prosthetic heart valve V. This is accomplished by fitting some or, preferably, all of the attachment loops 635 at the distal ends of tethers 615 over respective pins on prosthetic heart valve V. Although this is described here as an initial step, it need not be the first step in the process. Suture rigging assembly 600 may be attached to delivery device 100 first, as described below, followed by the attachment of prosthetic heart valve V to the suture rigging assembly 600.

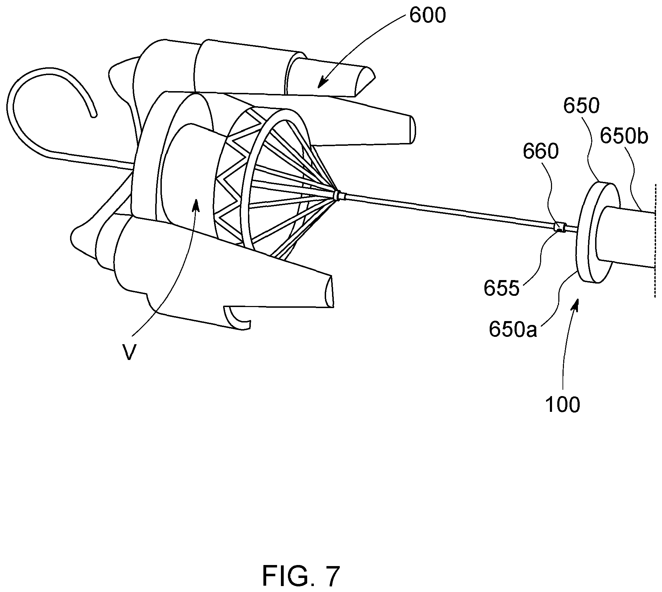

Referring to Fig. 7, a loading funnel 650 can be used to help load prosthetic heart valve V into valve cover . In one embodiment, loading funnel 650 may include a funnel portion 650a located at its distal end and an elongated tubular portion 650b located at its proximal end. Funnel portion 650a may smoothly transition from a relatively large diameter at its distal end to a relatively small diameter where it meets tubular portion 650b. The diameter at the distal end of funnel portion 650a is preferably larger than the outer diameter of prosthetic heart valve V in its expanded condition, and the diameter of the funnel portion where it meets tubular portion 650b is preferably about the same as the diameter of the lumen of the tubular portion 650b. The outer diameter of tubular portion 650b is preferably slightly smaller than the inner diameter of valve cover and the length of the tubular portion may be about the same as the length of the valve cover, such that the tubular portion can be selectively inserted into and nest within the valve cover. Funnel portion 650a may include a plurality of slots (not shown) extending longitudinally along its inner surface. These slots are intended to accommodate the pins of prosthetic heart valve V and prevent them from bending laterally as the heart valve is being collapsed.

With the tubular portion 650b of loading funnel 650 coupled to a distal end of the valve cover, controls located on the operating handle of delivery device 100 may be manipulated to cause suture catheter 655 to advance distally relative to the other components of the delivery device until the tip ring 660 of the suture catheter extends distally beyond the distal end of the tubular portion and into the interior of funnel portion 650a. At that point, the threads of coupling ring 601 may be threaded into the threaded portion of tip ring 660 at the distal end of suture catheter 655. Fig. 7 illustrates suture rigging assembly 600 connected to prosthetic heart valve V and aligned for connection to the suture catheter 655 of delivery device 100. Suture catheter 655 may then be retracted proximally, drawing suture rigging assembly 600 and prosthetic heart valve V proximally along with it. As proximal movement continues, tethers 615 are drawn into the lumen of the tubular portion 650b of loading funnel 650. This, in combination with the sloping walls of funnel portion 650a, causes the petals on atrial anchor of the prosthetic heart valve V to collapse toward the central axis of prosthetic heart valve V and, eventually, to enter the lumen of tubular portion 650b. Further proximal movement of suture catheter 655 continues until the petals on ventricular anchor also collapse toward the central axis of prosthetic heart valve V and the prosthetic heart valve is completely collapsed and completely positioned within the lumen of the tubular portion 650b. At that juncture, while maintaining tension on suture catheter 655, loading funnel 650 may be removed from valve cover, leaving the fully collapsed prosthetic heart valve V positioned completely within the valve cover. An atraumatic tip (not shown) of the delivery device may then be coupled to the nosecone catheter and then retracted to enclose the open distal end of valve cover.

In another embodiment, the loading funnel may have a generally cylindrical shape with internal threads at one end and an internal diameter that is about the same as the inner diameter of valve cover. The internal threads may mate with external threads at the free end of valve cover to join the loading funnel to the valve cover. A smooth radius on the lumen at the free end of the funnel may help to guide the prosthetic heart valve into the funnel lumen.

Once properly loaded, delivery device 100 may be inserted into a patient and directed to a target location, such as the mitral valve annulus, at which prosthetic heart valve V may be deployed. To deploy prosthetic heart valve V, valve cover is retracted proximally over valve V while the valve is maintained in position by the extension catheter (or the intermediate catheter). The ventricular anchor of valve V will then begin to expand until only the proximal end of the valve (*i.e.,* atrial anchor) is held in a collapsed condition by a small cup (*e.g.* valve retainer or can) at the distal end of extension catheter. The accurate positioning and orientation of prosthetic heart valve V may then be confirmed, after which suture catheter 655 may be advanced distally, relieving tension in tethers 615 and allowing atrial anchor to escape from the valve retainer at the distal end of extension catheter and expand. Suture catheter 655 may be advanced further through the expanded prosthetic heart valve until tethers 615 slip off of the pins of the frame of the prosthetic heart valve. Suture catheter 655 may then be retracted back into outer delivery sheath, the atraumatic tip may be retracted to again close the open end of valve cover, and delivery device 100 may be removed from the patient.

As noted above, catheters such as steering catheter 410a my incorporate tension cables to allow for deflection of the steering catheter. In some examples, steering can be provided using a single tension cable, without a corresponding return cable. In those examples, steering (which may also be referred to as catheter deflection) is performed by tensioning the single tension cable which has a distal end coupled to a distal portion of the steering catheter. After achieving deflection, there is no mechanism in these systems to actively return to a non-deflected state, rather relying on passive return to a non-deflected state. However, in other examples, including those shown and described above, steering can be provided using one or more pairs of tension cables, with one cable being tensioned to deflect the catheter, and the other cable of the pair being tensioned to actively return the catheter to a non-deflected state (or otherwise being tensioned to deflect the steering catheter in the opposite direction). In these types of systems, such as that shown in Fig. 4A, the tension cable of a steering cable pair is typically offset by about 180 degrees from the return cable of the steering cable pair, such that each cable provides for opposite directions of deflection. Each cable of the cable pair is also typically coupled to the same actuator (*e.g.,* knob, slider, gear) so that actuation of one of the cables of the pair simultaneously causes tensioning of one cable and relaxation of the other cable in the pair. It should also be noted that the 180 degree offset described above is not a requirement. For example, in the example shown in Fig. 4B, the tension cable of a steering cable pair may be offset by less than 180 degrees from the return cable of the steering cable pair. Even in this situation, although the tension cable and return cable are not exactly offset diametrically, the same general principles apply regarding the tension cable and the return cable being tensioned to deflect the catheter in generally opposite directions. Further details of these types of systems are provided in U.S.

### Patent Application Publication No. 2023/0364387.

Referring back to Figs. 1 and 4A (although this description generally applies to the example of Fig. 4B as well), one tension cable of a steering cable pair may extend through steering catheter 410a via lumens 421a, and the return cable of the steering cable pair may extend through the pair of lumens diametrically opposite the lumens labeled 412a. Similarly, a second tension cable of a second steering cable pair may extend through a third pair of the lumens, and a second return cable of the second steering cable pair may extend through a fourth pair of the lumens diametrically opposite the third pair. In some examples, only a single lumen per steering cable is used, with the proximal end of the steering cable connected to a steering actuator at the delivery device handle, and the distal end of the steering cable connected to a distal end portion of the steering catheter, for example via a steering ring. However, in other examples, including that illustrated in Fig. 4A, a single steering cable extends distally through one lumen 412a of the lumen pair, connects to the steering ring and loops back through the other lumen 412a of the lumen pair. This type of configuration may provide greater strength to the steering cable compared to the single cable - single lumen example.

One issue that can occur in steering catheters that utilize cable pairs (e.g. one tension cable and one return cable per steering direction) is that either cable of the pair may have some amount of slack in the cable before being actuated. In this situation, if a user actuates a steering actuator (e.g., knob) to tension a cable of the cable pair to cause catheter deflection, but the cable that is to be tensioned includes some slack at the moment of actuation, a first amount of the actuation (e.g., an initial amount of rotation of the knob) will cause the cable to lose its slack. As the actuator causes the cable to lose its slack, the catheter does not actually begin to deflect until the slack is removed and continued actuation of the actuation (e.g., continued rotation of the knob) applies tension to the steering cable that actually gets transmitted to the steering catheter (instead of just working to reduce the slack). The range of actuation in which tension on the cable only works to reduce slack, instead of cause the desired steering, may be referred to as a control deadband where, although the user is actuating the actuator, no steering actually results from the actuator. Stated in other words, if there is slack in the steering cable just before actuation, rotating the steering knob (or actuating the steering actuator generally) results in a non-responsive control while the slack in the steering cable is taken up. Thus, it would be preferable to reduce or eliminate the likelihood of slack that may occur in steering cables, thus reducing or eliminating the control deadband perceived by the operator during steering operations.

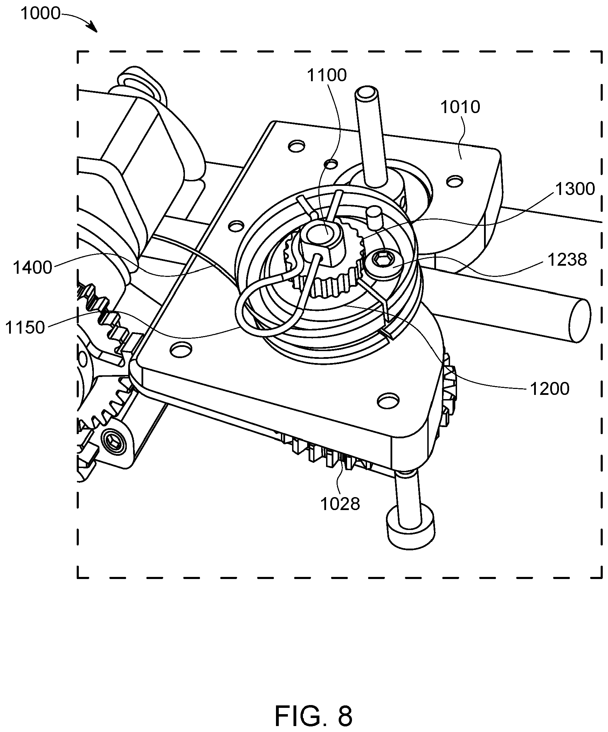

Figs. 8-10 show various views of internal components of internal components of a steering assembly 1000 that may provide benefits over traditional catheter steering assemblies. Although a single steering assembly 1000 is shown and described, it should be understood that more than one of the steering assemblies may be provided with a catheter system. For example, up to three of the steering assemblies 1000 may be provided for use with delivery system 100 shown and described above, for example within handle assembly 105, with one steering knob 110 or steering actuator corresponding to each of the three steering assemblies 1000 positioned inside of the handle assembly 105. However, it should be understood that the concepts and features described in connection with steering assembly 1000 may be applied to any catheter system with steering capabilities, particularly those including bi-directional steering.

Figs. 8-10 illustrate different views of an example of a steering assembly 1000, although it should be noted that components of another steering assembly are also visible in the figures. The steering assembly 1000 may include a base 1010 which may generally provide support for other components that are attached directly or indirectly to the base 1010 to form the steering assembly 1000. Although the base 1010 has one particular form factor in the figures, it should be understood that any other suitable form factor may be used to house and/or support the various other components of the steering assembly 1000.

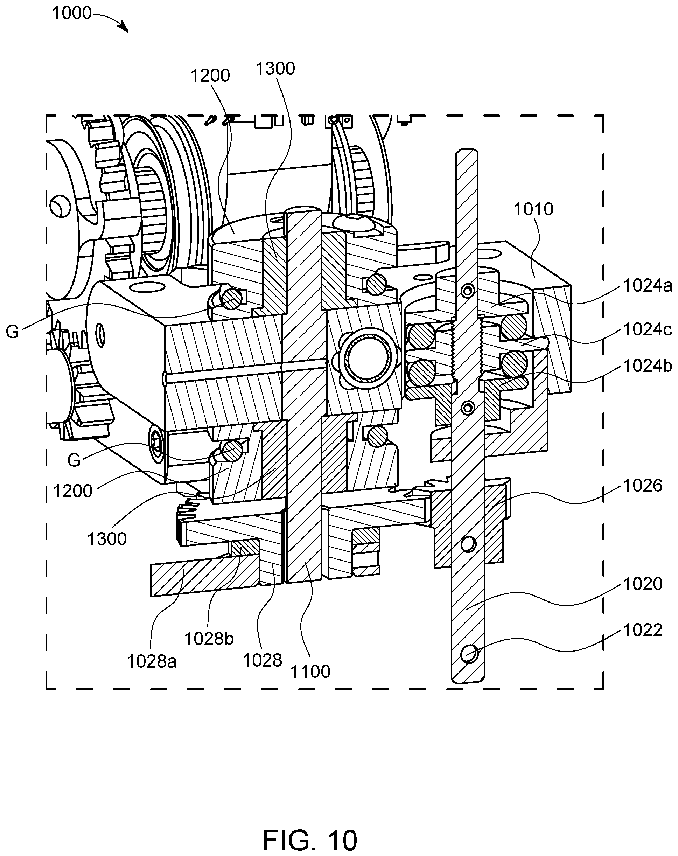

Referring now to Fig. 10, the steering assembly 1000 may include an actuator shaft 1020 which may generally take the form of a cylindrical member. A first end of the actuator shaft may include an aperture 1022. In one example, the aperture 1022 may be configured to receive a shaft of an actuator, such as a shaft of a steering knob 110, such that rotation of the knob 110 in either rotational direction results in corresponding rotation of the actuator shaft 1020. The actuator shaft 1020 may extend from the first end to a second opposite end, with a middle portion thereof extending through the base 1010. For example, a mid-portion of the actuator shaft 1020 may be coupled to and extend through one or more supports 1024a-b, which may include bearings or other components to help secure the actuator shaft 1020 relative to the base 1010 and to assist with the rotation of the actuator shaft 1020 relative to the base 1010. For example, a top support 1024a may be generally cylindrical and fixed to the actuator shaft 1020, a bottom support 1024b may be generally cylindrical and fixed to the actuator shaft 1020, and a center support 1024c may be positioned between the top and bottom supports 1024a-b, with one or more bearings (*e.g.* ball bearings) being positioned between the top support 1024a and the center support 1024c, as well as between the bottom support 1024b and the center support 1024c.

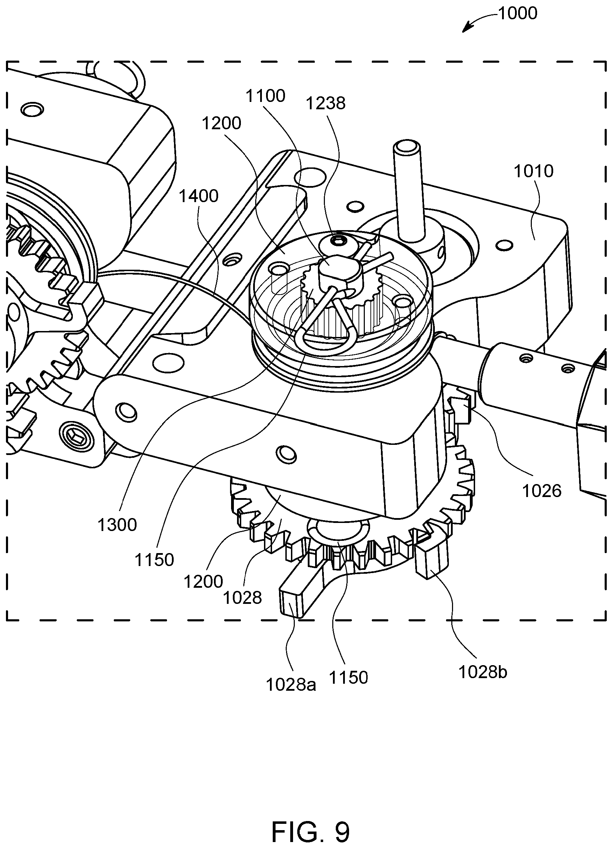

Still referring to Fig. 10, the actuator shaft 1020 may be fixed to a knob gear wheel 1026 (a portion of which is also visible in Fig. 9), which may be a relatively small gear with teeth that mesh with teeth of a relatively large drive gear 1028. With this configuration, rotation of an actuator (*e.g.* steering knob 110) coupled to actuator shaft 1020 causes rotation of knob gear wheel 1026, and thus rotation of drive gear 1028, to activate a steering operation, described in further detail below.

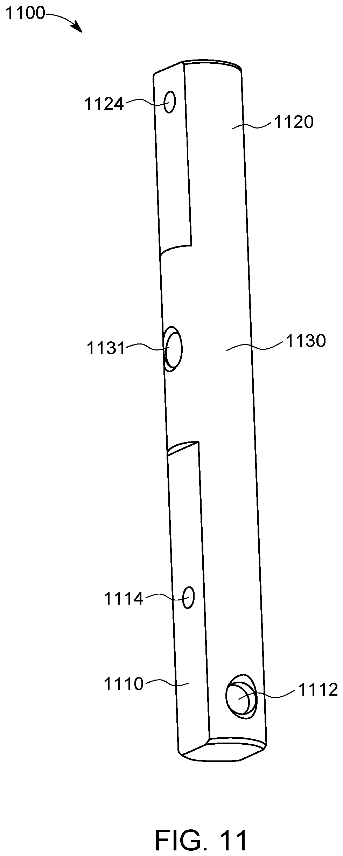

Referring mainly to Figs. 9-10, drive gear 1028 may take the form of a toothed gear wheel with a central opening. The central opening of the drive gear 1028 in the illustrated embodiment has a cross-sectional shape with two opposing flats and two rounded sections connecting the pair of flats. In other words, the central opening of the drive gear 1028 may be stadium-shaped in some embodiments, although in other embodiments any shape that permits torque translation (*e.g.* triangular, rectangular, etc.) may be suitable. Referring to Figs. 10-11, the central opening of the drive gear 1028 may receive a bottom end portion 1110 of a spool shaft 1100. As shown in Fig. 11, the bottom end portion 1110 of spool shaft 1100 may have a complementary shape to the central opening of the drive gear 1028, including for example a general stadium shape with two opposing flats and rounded portions connecting the pair of flats. With this configuration, during assembly, the spool shaft 1100 may only be inserted into the drive gear 1028 in two possible orientations, either of which is acceptable and both of which result in effective torque transmission between the spool shaft 1100 and the drive gear 1028. The bottom end portion 1110 of the spool shaft 1100 may include a through bore 1112, although in other embodiments through bore 1112 may be omitted. If included, through bore 1112 may allow the spool shaft 1100 to be used for either a right-hand or left-hand user, for example my reversing the orientation the spool shaft 1100 and using through bore 1112 for receiving a pin 1150 instead of using through bore 1114 (described in greater detail below).

Still referring to Figs. 9-10, the drive gear 1028 may include a small diameter extension extending below the gear teeth. One or more hard stop features may be provided on the drive gear 1028. For example, a first hard stop 1028a may be received over the small diameter extension of the drive gear 1028, with the first hard stop 1028a including an extension that rotates along with the drive gear 1028. When the first hard stop 1028a contacts another component, *e.g.* the smooth body of knob gear wheel 1026, the contact will prevent further rotation of the drive gear 1028, providing for a limit of rotation in at least one rotational direction. A second hard stop 1028b may also be provided. For example, as shown in Fig. 9, second hard stop 1028b may include a hooked end. In the illustrated example, while the first hard stop 1028a is positioned below the plane of the drive gear 1028, the second hard stop 1028b is positioned generally coplanar with the plane of the drive gear 1028. However, in some embodiments, part of the second hard stop 1028b may be coupled to the drive gear 1028 below the plane the teeth of the drive gear 1028, with a portion of the hard stop 1028b (*e.g.* the hooked portion) being coplanar with the plane of the drive gear 1028. With this configuration, as the drive gear 1028 is rotated in the direction of the hooked end of the second hard stop 1028b, the hooked end will eventually intermesh with gear teeth of the knob gear wheel 1028, binding the gears and serving as another limit of rotation. In some embodiments, the second hard stop 1028b may serve as a rotation limiter in one rotational direction (e.g. clockwise) while the first hard stop 1028a may serve as a rotation limiter in the other rotational direction (e.g. anti-clockwise).

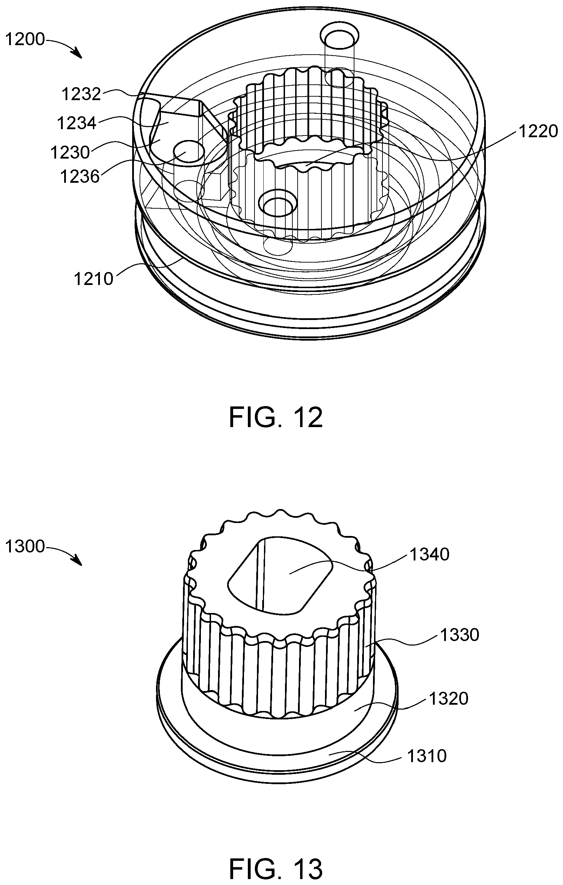

Referring to Fig. 10, two similar or identical spool assemblies are shown being coupled to the spool shaft 1100 on opposite sides of the base 1010. As will described in greater detail below, each spool assembly may be coupled to a steering wire (which may also be referred to as a pull-wire) of a pair of steering wires configured to provide bi-directional steering (*e.g.,* deflection in opposite directions along the same steering direction or steering plane). Each spool assembly may include an outer spool 1200 (which may alternatively be referred to simply as a spool) and an inner spool 1300 (which may alternatively be referred to as an inner spline or a spline nut), with these components being shown in Figs. 12 and 13, respectively, in isolation.

Figs. 8-10 show a first or upper spool assembly positioned on the opposite side of the base 1010 as the drive gear 1028. Fig. 8 shows that a steering cable 1400 may extend from the outer spool 1200 where it passes into the steering catheter. For example, the steering cable 1400 may have a first portion that extends through one lumen 412a of a lumen pair of steering catheter 410a, distally down the steering catheter until it loops around a steering ring, and then proximally back through an adjacent lumen 412a of the lumen pair, eventually returning to the outer spool 1200. However, in other embodiments, as mentioned above, the steering cable 1400 may have a first end that terminates at the outer spool 1200 and a second end that terminates at the steering ring, without the looped configuration described above. As shown in in Fig. 12, the outer spool 1200 may include a recess in the form of a cable track 1210 which extends a distance radially inwardly toward an interior cylindrical column that generally surrounds the spline recess 1220, described in greater detail below. This interior cylindrical column may have an outer diameter that is smaller than the outer diameter of the portions of the outer spool 1200 above and below the cable track 1210. The steering wire 1400 may extend into the cable track 1210, wrap at least partially around the interior cylindrical column, and terminate at a connection location 1230. Referring in particular to Figs. 8 and 12, the connection location 1230 may include a recess 1232 that may generally extend downwardly to connect to cable track 1210, so that the steering cable 1400 can be routed from the cable track 1210, up the recess 1232, and then into an enlarged area 1234 of the connection location 1230. The connection location 1230 may include a through bore 1236 in the enlarged area 1234, the through bore 1236 configured to receive a fastener such as a screw 1238, shown in Fig. 8. The fastener 1238 may be fixedly coupled to the outer spool 1200 via the through bore 1236, and an end of the steering cable 1400 may be coupled to the outer spool 1200 via the fastener 1238. For example, the end(s) of the steering cable 1400 may be received in the enlarged area 1234 and clamped down via a head of the screw or fastener 1238 that is engaged with the through bore 1236. With this configuration, as will be described in greater detail below, as the outer spool 1200 rotates, the fixed coupling between the steering cable 1400 and the fastener 1238 ensures that the steering cable 1400 can be tensioned while the cable track 1210 guides the portions of the steering cable 1400 that are being drawn into the outer spool 1200 and wound around the inner cylinder of the outer spool 1200.

Each spool assembly, including the upper spool assembly best shown in Figs. 8-10, may also include an inner spool 1300 received within the spline recess 1220 of the outer spool 1200. Referring to Fig. 13, each inner spool 1300 may include a relatively thin bottom flange 1310 having a relatively large diameter, that transitions to a center column section which may include a smooth base 1320 having a smaller diameter than the flange 1310, and a spline section 1330 extending upwardly from the smooth base 1320. Although the steering cable is generally configured to wrap around the track 1210 of the outer spool 1200, a portion of the innermost loop of the steering cable may come into contact with smooth base 1320. In some examples, the spline section 1330 is taller than both the smooth base 1320 and the flange 1310. The spline section 1330 may include a plurality of splines, which may also be referred to as alternating peaks or ridges and valleys or recesses which all extend in a longitudinal direction (*e.g.,* parallel to the axis of rotation). The number of splines in the spline section 1330 may be more than or fewer than shown in Fig. 13, and the number may be increased or decreased to provide more or less (respectively) resolution of pre-tension on the steering cable 1400, which is described in greater detail below. Notably, the spline section 1330 preferably has a number and configuration of splines that is complementary to the shape of the radially-inner surface of the spline recess 1220 of the outer spool so that each ridge of the spline section 1330 may be received within a corresponding valley of the spline recess 1220, and each ridge of the spline recess 1220 may be received within a corresponding valley of the spline section 1330. The inner spool 1300 may also include a central longitudinal recess 1340 that has a stadium-shape or another torque-transmitting shape that is complementary to the shapes of the bottom end portion 1110 and the top end portion 1120 of the spool shaft 1100.

Referring to Figs. 8-11, the spool shaft 1100 may include a cylindrical central portion 1130 between the stadium-shaped top end portion 1120 and bottom portion 1110, and as best shown in Fig. 10, the central portion 130 may be received within a cylindrical through bore of the base 1010, such that the top end portion 1120 protrudes above the base 1010 and the bottom end portion 1110 protrudes below the base 1010. In this configuration, one inner spool 1300 may be slid over the top end portion 1120 of the spool shaft 1100, and another inner spool 1300 may be slid over the bottom end portion 1110 of the spool shaft 1100, with the two inner spools 1300 both having their flanges 1310 in contact with the base 1010. At this point, one outer spool 1200 may be slid over the top inner spool 1300, and another outer spool 1200 may be slid over the bottom inner spool 1300. When the inner spool 1300 is received within the outer spool 1200, the splines of the spline section 1330 engage the corresponding splines of the spline recess 1220, so that the inner spool 1300 is rotationally locked to the outer spool 1200. Referring to Figs. 10-11, the spool shaft 1100 may include a central aperture 1131 that can align with a lumen of the base 1010 (as shown in Fig. 10). During assembly, a pin or other device may be temporarily inserted through the lumen of the base and into the central aperture 1131 to assist with locking rotation of the spool shaft 1100 during assembly. After assembly is completed, the pin may be removed from the lumen and the central aperture 1131 to again allow for rotation of the spool shaft 1100 relative to the base 1010.

After the outer spools 1200 are received over the inner spools 1300, and the inner spools 1300 are received over the spool shaft 1100, coupling members may be used to lock the axial positions of the spool assemblies to the spool shaft 1100. For example, referring to Fig. 11, a through bore 1114 may be provided in the bottom end section 1110 of the spool shaft 1100, and another through bore 1124 may be provided in the top end section 1120 of the spool shaft. As best shown in Figs. 8-9, these through bores 1114, 1124 may be configured to be positioned just beyond the top and bottom ends of the spool assemblies, respectively, so that one pin 1150 or a similar device (*e.g.* a cotter pin) can be passed through each of the through bores 1114, 1124, ensuring the spool assemblies are axially locked between the pin 1150 and the base 1010. At this point, the drive gear 1028 may be positioned over the bottom end portion 1110 of the spool shaft 1100 (which may be longer than the top end portion 1120 so that there is enough space to accommodate the drive gear 1028), and assembly of the steering assembly 1000 may be substantially complete once the drive gear 1028 is fixed to the spool shaft 1100 and intermeshes with the knob gear wheel 1026. As noted above, the steering assembly 1000 may be encased within a housing which the actuator shaft 1020 protrude from so that an actuator, such as steering knob 110, may be fixed to the actuator shaft 1020.

Although Fig. 8 only shows the top spool assembly with the steering cable 1400 being coupled to the outer spool 1200 via fastener 1238 and extending toward the steering catheter, it should be understood that the bottom spool assembly may have an identical configuration except that the steering cable coupled to the bottom spool assembly may be wound about the spool in an opposite direction compared to the top spool assembly. Further, it should be understood that the steering cable coupled to the bottom spool assembly may extend toward the steering catheter and into a pair of lumens (*e.g.* a pair of lumens 412a) which may be substantially diametrically opposed to the pair of lumens through which the top steering cable 1400 extends. However, as explained above, in some examples, particularly in those with three or more pairs of steering cable lumens, the two opposing tension/return cables corresponding to a single bi-directional steering direction need not be exactly diametrically opposed, and rather may be slightly offset from being diametrically opposed.

With the configuration described above, when actuator shaft 1020 is rotated in a first direction (*e.g.,* by rotating steering knob 110 in the first direction), the knob gear wheel 1026 rotates in the first direction, causing the drive gear 1028 to rotate in the opposite direction. As the drive gear 1028 rotates, it causes the spool shaft 1100 to rotate due to the corresponding torque-transmitting shapes of center opening in the drive gear 1028 and the bottom end portion 1110 of the spool shaft 1100. As the spool shaft 1100 rotates, it causes both inner spools 1300 to rotate in the same direction, again due to the corresponding torque-transmitting shapes of the top and bottom end portions 1120, 1110 of the spool shaft 1100 and that of the longitudinal recess 1340 of the inner spool 1300. As the inner spool 1300 rotates, the engagement between the splines of the spline section 1330 of the inner spool 1300 with the interior splines of the spline recess 1220 causes the outer spool 1200 to rotate along with the inner spool 1300. As the outer spools rotate 1200 in the same direction as each other, one spool will tend to tension its connected steering cable as the steering cable winds around the spool, while the other spool will tend to slacken its connected steering cable as the steering cable unwinds from the spool, because as noted above, the steering cables are wound in opposite directions along the two spools. Thus, actuating the actuator shaft 1020 (*e.g.* by rotating steering knob 110) in a first direction will tension one steering cable of the pair and simultaneously slacken the other steering cable of the pair to cause bending of the steering catheter in a first direction, while actuating the actuator shaft 1020 in a second direction opposite the first direction will have the opposite effect, causing bending of the steering catheter in a second direction opposite the first direction. Thus, bidirectional steering may be achieved with a single steering knob 110. Referring briefly to Fig. 10, each outer spool 1200 may include a gasket G that generally overlies a part of the relevant steering cable so that, as slack is introduced into the steering cable, the steering cable generally remains within the track 1210 of the outer spool 1200.

As explained above, one benefit of the configuration shown and described in connection with Figs. 8-13 is that a single steering actuator may be manipulated to cause simultaneous tensioning (by winding around one spool) of one steering cable of a pair and slackening (by unwinding around another spool) of the other steering cable of a pair to achieve bidirectional steering with a single steering wheel (or knob, slider, *etc.*)*.*

However, another benefit of this configuration is that it allows for substantial or complete elimination of a perceived control deadband due to slack existing in steering cables just prior to actuating the steering mechanism. For example, during assembly of the outer spool 1200 (to which the end(s) of the steering cable is fixed), the outer spool 1200 can be situated over the inner spool 1300 in a plurality of different rotational orientations. For example, referring to Figs. 12-13, if the spline section 1330 includes twenty splines (and the spline recess 1220 includes a corresponding twenty splines), there are twenty unique rotational positions that the inner spool 1300 may have relative to the outer spool 1200, with each rotational position changing in increments of about eighteen degrees. It should be understood that the example of twenty splines is merely exemplary, and more splines may be included to provide even more unique rotational positions (with smaller degrees of offset between different positions) or fewer splines may be included to provide for fewer unique rotational positions (with larger degrees of offset between different positions). With this configuration, when the outer spool 1200 is being slipped over the inner spool 1300 to form the spool assembly, the user may choose between these various unique rotational positions, which may allow the user to fine tune how much tension is on the steering cable at the time of assembly, which may be referred to as pre-tensioning. Thus, pre-tension on the steering cables may be set during assembly, with the desired amount of pre-tension being determined by which incremental rotational position the outer spool 1200 has relative to the inner spool 1300 at assembly. This can be performed for both spool assemblies, so that both steering cables have a desired amount of pre-tension. By pre-tensioning the steering cables, neither steering cable will have slack (or meaningful slack) when the knob 110 (or other steering actuator) is actuated to rotate the actuator shaft 1020 to deflect the steering catheter in the desired direction, and thus any perceived control deadband will be fully (or substantially fully) eliminated. Further, because actuation of the actuator shaft 1020 causes simultaneously winding of one steering cable around its spool and unwinding of the other steering cable around its spool, slack is not likely to be introduced into either steering cable at any time during the procedure.

Although the steering assemblies herein are described in general terms of a steering catheter for delivering a prosthetic heart valve, it should be understood that these concepts and/or features may be implemented in any steering catheter without needing significant alteration of the components. Further, although the steering assemblies are described in connection with bi-directional steering (*e.g.,* with two spool assemblies controlling the tension and return directions), at least some of the benefits described herein may be achieved with unidirectional steering (*e.g.,* with one spool assembly only controlling only a single direction of steering).

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the appended claims.

## Claims

1. A catheter system (100) comprising:
a steering catheter (410a, 410b) having a wall and a first lumen (412a, 412b) formed within the wall;
a first steering cable (1400) extending through the first lumen along a length of the wall of the steering catheter toward a distal end portion of the steering catheter, the first steering cable being coupled to the distal end portion of the steering catheter; and
a steering assembly (1000) including a first spool assembly, the first spool assembly including a first outer spool (1200), a first inner spool (1300) received at least partially within a recess (1220) of the first outer spool, and a spool shaft (1100) having a first end portion (1120) received at least partially within a recess (1340) of the first inner spool,
wherein the first end portion of the spool shaft and the recess of the first inner spool are each shaped so that rotation of the spool shaft about a longitudinal axis thereof transmits torque to the first inner spool to cause rotation of the first inner spool about the longitudinal axis of the spool shaft;
wherein the first inner spool includes a spline section (1330) forming a plurality of splines, and the recess of the first outer spool has a shape that is complementary to the plurality of splines, such that when the first inner spool is received at least partially within the recess of the first outer spool, the plurality of splines engage the complementary shape of the recess of the first outer spool to prevent rotation of the first inner spool relative to the first outer spool about the longitudinal axis of the spool shaft; and
wherein the first steering cable is fixed to the first outer spool and configured to be wound around the first spool assembly upon rotation of the first outer spool about the longitudinal axis.

2. The catheter system (100) of claim 1, wherein first inner spool (1300) is configured to be received within the first outer spool (1200) in any one of a plurality of unique relative rotational positions, so that the first steering cable (1400) is configured to have a first amount of pretension in a first one of the plurality of unique relative rotational positions and a second amount of pretension in a second one of the plurality of unique relative rotational positions, the first amount of pretension being different than the second amount of pretension.

3. The catheter system (100) of claim 1 or 2, wherein the steering assembly (1000) includes a base (1010), the spool shaft (1100) has a central portion (1130) received within the base, and the first spool assembly is positioned on a first surface of the base.

4. The catheter system (100) of claim 3, wherein the steering assembly (1000) includes a second spool assembly positioned on a second surface of the base (1010) opposite the first surface of the base.

5. The catheter system (100) of claim 4, wherein the second spool assembly includes a second outer spool (1200), and a second inner spool (1300) received at least partially within a recess (1220) of the second outer spool.

6. The catheter system (100) of claim 5, wherein the first inner spool (1300) is identical to the second inner spool (1300), and the first outer spool (1200) is identical to the second outer spool (1200).

7. The catheter system (100) of claim 5, wherein the spool shaft (1100) has a second end portion (1110) received at least partially within a recess (1340) of the second inner spool (1300), and the second end portion of the spool shaft and the recess of the second inner spool are each shaped so that rotation of the spool shaft about the longitudinal axis thereof transmits torque to the second inner spool to cause rotation of the second inner spool about the longitudinal axis of the spool shaft.

8. The catheter system (100) of claim 7, wherein the second inner spool (1300) includes a spline section (1330) forming a plurality of splines, and the recess (1220) of the second outer spool (1200) has a shape that is complementary to the plurality of splines of the spline section of the second inner spool, such that when the second inner spool is received at least partially within the recess of the second outer spool, the plurality of splines of the spline section of the second inner spool engage the complementary shape of the recess of the second outer spool to prevent rotation of the second inner spool relative to the second outer spool about the longitudinal axis of the spool shaft.

9. The catheter system (100) of claim 8, further comprising:
a second lumen (412a, 412b) formed within the wall of the steering catheter (410a, 410b); and
a second steering cable (1400) extending through the second lumen along a length of the wall of the steering catheter toward the distal end portion of the steering catheter, the second steering cable being coupled to the distal end portion of the steering catheter,
wherein the second steering cable is fixed to the second outer spool (1200) and configured to be wound around the second spool assembly upon rotation of the second outer spool about the longitudinal axis.

10. The catheter system (100) of claim 9, wherein the first steering cable (1400) is fixed to the first outer spool (1200) and the second steering cable (1400) is fixed to the second outer spool (1200) such that, when the spool shaft (1100) is rotated about the longitudinal axis to cause simultaneous rotation of the first outer spool and the second outer spool, the first steering cable winds around the first spool assembly while the second steering cable unwinds from the second spool assembly.

11. The catheter system (100) of claim 10, wherein the first lumen (412a, 412b) is positioned on a generally opposite side of the steering catheter (410a, 410b) compared to the second lumen (412a, 412b).

12. The catheter system (100) of claim 11, wherein tensioning the first steering cable (1400) is configured to deflect the distal end portion of the steering catheter (410a, 410b) in a first steering direction, and tensioning the second steering cable (1400) is configured to deflect the distal end portion of the steering catheter in a second steering direction, the first steering direction being generally opposite the second steering direction.

13. The catheter system (100) of claim 10, wherein the first lumen (412a, 412b) is positioned diametrically opposite to the second lumen (412a, 412b).

14. The catheter system (100) of claim 13, wherein tensioning the first steering cable (1400) is configured to deflect the distal end portion of the steering catheter (410a, 410b) in a first steering direction, and tensioning the second steering cable (1400) is configured to deflect the distal end portion of the steering catheter in a second steering direction, the first steering direction and the second steering direction being positioned in a same plane but in opposite directions along the same plane.

15. The catheter system (100) of claim 10, further comprising a drive gear (1028), the drive gear having a central opening, the second end portion (1110) of the spool shaft (1130) being received within the central opening, wherein the second end portion of the spool shaft and the central opening of the drive gear are each shaped so that rotation of the drive gear about the longitudinal axis of the spool shaft transmits torque to the spool shaft to cause rotation of the spool shaft about the longitudinal axis of the spool shaft.

## Patentansprüche

1. Kathetersystem (100), aufweisend:
einen Lenkkatheter (410a, 410b) mit einer Wand und einem ersten Lumen (412a, 412b), das in der Wand ausgebildet ist;
ein erstes Lenkkabel (1400), das sich durch das erste Lumen entlang einer Länge der Wand des Lenkkatheters zu einem distalen Endabschnitt des Lenkkatheters erstreckt, wobei das erste Lenkkabel mit dem distalen Endabschnitt des Lenkkatheters gekoppelt ist; und
eine Lenkanordnung (1000) mit einer ersten Spulenanordnung, wobei die erste Spulenanordnung eine erste äußere Spule (1200), eine erste innere Spule (1300), die zumindest teilweise in einer Aussparung (1220) der ersten äußeren Spule aufgenommen ist, und eine Spulenwelle (1100) mit einem ersten Endabschnitt (1120), der zumindest teilweise in einer Aussparung (1340) der ersten inneren Spule aufgenommen ist, aufweist,
wobei der erste Endabschnitt der Spulenwelle und die Aussparung der ersten inneren Spule jeweils so geformt sind, dass eine Drehung der Spulenwelle um eine Längsachse davon ein Drehmoment auf die erste innere Spule überträgt, um eine Drehung der ersten inneren Spule um die Längsachse der Spulenwelle zu bewirken;
wobei die erste innere Spule einen Keilabschnitt (1330) aufweist, der eine Mehrzahl von Keilen bildet, und die Aussparung der ersten äußeren Spule eine Form aufweist, die zu der Mehrzahl von Keilen komplementär ist, so dass, wenn die erste innere Spule zumindest teilweise in der Aussparung der ersten äußeren Spule aufgenommen ist, die Mehrzahl von Keilen in die komplementäre Form der Aussparung der ersten äußeren Spule eingreift, um eine Drehung der ersten inneren Spule relativ zu der ersten äußeren Spule um die Längsachse der Spulenwelle zu verhindern; und
wobei das erste Lenkkabel an der ersten äußeren Spule befestigt ist und konfiguriert ist, um bei einer Drehung der ersten äußeren Spule um die Längsachse um die erste Spulenanordnung gewickelt zu werden.

2. Das Kathetersystem (100) nach Anspruch 1, wobei die erste innere Spule (1300) konfiguriert ist, um in der ersten äußeren Spule (1200) in einer beliebigen einer Mehrzahl von eindeutigen relativen Drehpositionen aufgenommen zu werden, so dass das erste Lenkkabel (1400) konfiguriert ist, um einen ersten Betrag an Vorspannung in einer ersten der Mehrzahl von eindeutigen relativen Drehpositionen und einen zweiten Betrag an Vorspannung in einer zweiten der Mehrzahl von eindeutigen relativen Drehpositionen aufzuweisen, wobei sich der erste Betrag an Vorspannung von dem zweiten Betrag an Vorspannung unterscheidet.

3. Das Kathetersystem (100) nach Anspruch 1 oder 2, wobei die Lenkanordnung (1000) eine Basis (1010) aufweist, die Spulenwelle (1100) einen zentralen Abschnitt (1130) aufweist, der in der Basis aufgenommen ist, und die erste Spulenanordnung auf einer ersten Oberfläche der Basis positioniert ist.

4. Das Kathetersystem (100) nach Anspruch 3, wobei die Lenkanordnung (1000) eine zweite Spulenanordnung aufweist, die auf einer zweiten Oberfläche der Basis (1010) gegenüber der ersten Oberfläche der Basis positioniert ist.

5. Das Kathetersystem (100) nach Anspruch 4, wobei die zweite Spulenanordnung eine zweite äußere Spule (1200) und eine zweite innere Spule (1300) aufweist, die zumindest teilweise in einer Aussparung (1220) der zweiten äußeren Spule aufgenommen ist.

6. Das Kathetersystem (100) nach Anspruch 5, wobei die erste innere Spule (1300) mit der zweiten inneren Spule (1300) identisch ist und die erste äußere Spule (1200) mit der zweiten äußeren Spule (1200) identisch ist.

7. Das Kathetersystem (100) nach Anspruch 5, wobei die Spulenwelle (1100) einen zweiten Endabschnitt (1110) aufweist, der zumindest teilweise in einer Aussparung (1340) der zweiten inneren Spule (1300) aufgenommen ist, und der zweite Endabschnitt der Spulenwelle und die Aussparung der zweiten inneren Spule jeweils so geformt sind, dass eine Drehung der Spulenwelle um die Längsachse davon ein Drehmoment auf die zweite innere Spule überträgt, um eine Drehung der zweiten inneren Spule um die Längsachse der Spulenwelle zu bewirken.

8. Das Kathetersystem (100) nach Anspruch 7, wobei die zweite innere Spule (1300) einen Keilabschnitt (1330) aufweist, der eine Mehrzahl von Keilen bildet, und die Aussparung (1220) der zweiten äußeren Spule (1200) eine Form aufweist, die zu der Mehrzahl von Keilen des Keilabschnitts der zweiten inneren Spule komplementär ist, so dass, wenn die zweite innere Spule zumindest teilweise in der Aussparung der zweiten äußeren Spule aufgenommen ist, die Mehrzahl von Keilen des Keilabschnitts der zweiten inneren Spule in die komplementäre Form der Aussparung der zweiten äußeren Spule eingreift, um eine Drehung der zweiten inneren Spule relativ zu der zweiten äußeren Spule um die Längsachse der Spulenwelle zu verhindern.

9. Das Kathetersystem (100) nach Anspruch 8, ferner aufweisend:
ein zweites Lumen (412a, 412b), das in der Wand des Lenkkatheters (410a, 410b) ausgebildet ist; und
ein zweites Lenkkabel (1400), das sich durch das zweite Lumen entlang einer Länge der Wand des Lenkkatheters zu dem distalen Endabschnitt des Lenkkatheters erstreckt, wobei das zweite Lenkkabel mit dem distalen Endabschnitt des Lenkkatheters gekoppelt ist,
wobei das zweite Lenkkabel an der zweiten äußeren Spule (1200) befestigt ist und konfiguriert ist, um bei einer Drehung der zweiten äußeren Spule um die Längsachse um die zweite Spulenanordnung gewickelt zu werden.

10. Das Kathetersystem (100) nach Anspruch 9, wobei das erste Lenkkabel (1400) an der ersten äußeren Spule (1200) befestigt ist und das zweite Lenkkabel (1400) an der zweiten äußeren Spule (1200) befestigt ist, so dass, wenn die Spulenwelle (1100) um die Längsachse gedreht wird, um eine gleichzeitige Drehung der ersten äußeren Spule und der zweiten äußeren Spule zu bewirken, sich das erste Lenkkabel um die erste Spulenanordnung wickelt, während sich das zweite Lenkkabel von der zweiten Spulenanordnung abwickelt.

11. Das Kathetersystem (100) nach Anspruch 10, wobei das erste Lumen (412a, 412b) auf einer im Allgemeinen gegenüberliegenden Seite des Lenkkatheters (410a, 410b) im Vergleich zu dem zweiten Lumen (412a, 412b) positioniert ist.

12. Das Kathetersystem (100) nach Anspruch 11, wobei das Spannen des ersten Lenkkabels (1400) konfiguriert ist, um den distalen Endabschnitt des Lenkkatheters (410a, 410b) in eine erste Lenkrichtung abzulenken, und das Spannen des zweiten Lenkkabels (1400) konfiguriert ist, um den distalen Endabschnitt des Lenkkatheters in eine zweite Lenkrichtung abzulenken, wobei die erste Lenkrichtung im Allgemeinen der zweiten Lenkrichtung entgegengesetzt ist.

13. Das Kathetersystem (100) nach Anspruch 10, wobei das erste Lumen (412a, 412b) diametral gegenüber dem zweiten Lumen (412a, 412b) positioniert ist.

14. Das Kathetersystem (100) nach Anspruch 13, wobei das Spannen des ersten Lenkkabels (1400) konfiguriert ist, um den distalen Endabschnitt des Lenkkatheters (410a, 410b) in eine erste Lenkrichtung abzulenken, und das Spannen des zweiten Lenkkabels (1400) konfiguriert ist, um den distalen Endabschnitt des Lenkkatheters in eine zweite Lenkrichtung abzulenken, wobei die erste Lenkrichtung und die zweite Lenkrichtung in einer gleichen Ebene, aber in entgegengesetzten Richtungen entlang der gleichen Ebene positioniert sind.

15. Das Kathetersystem (100) nach Anspruch 10, ferner aufweisend ein Antriebszahnrad (1028), wobei das Antriebszahnrad eine zentrale Öffnung aufweist, wobei der zweite Endabschnitt (1110) der Spulenwelle (1130) in der zentralen Öffnung aufgenommen ist, wobei der zweite Endabschnitt der Spulenwelle und die zentrale Öffnung des Antriebszahnrads jeweils so geformt sind, dass eine Drehung des Antriebszahnrads um die Längsachse der Spulenwelle ein Drehmoment auf die Spulenwelle überträgt, um eine Drehung der Spulenwelle um die Längsachse der Spulenwelle zu bewirken.

## Revendications

1. Système de cathéter (100) comprenant :
un cathéter de direction (410a, 410b) ayant une paroi et une première lumière (412a, 412b) formée à l'intérieur de la paroi ;
un premier câble de direction (1400) s'étendant à travers la première lumière le long d'une longueur de la paroi du cathéter de direction vers une partie d'extrémité distale du cathéter de direction, le premier câble de direction étant couplé à la partie d'extrémité distale du cathéter de direction ; et
un ensemble de direction (1000) comprenant un premier ensemble de bobine, le premier ensemble de bobine comprenant une première bobine externe (1200), une première bobine interne (1300) reçue au moins partiellement à l'intérieur d'un évidement (1220) de la première bobine externe, et un arbre de bobine (1100) ayant une première partie d'extrémité (1120) reçue au moins partiellement à l'intérieur d'un évidement (1340) de la première bobine interne,
dans lequel la première partie d'extrémité de l'arbre de bobine et l'évidement de la première bobine interne sont chacun formés de sorte que la rotation de l'arbre de bobine autour d'un axe longitudinal de celui-ci transmette un couple à la première bobine interne pour provoquer la rotation de la première bobine interne autour de l'axe longitudinal de l'arbre de bobine ;
dans lequel la première bobine interne comprend une section cannelée (1330) formant une pluralité de cannelures, et l'évidement de la première bobine externe a une forme qui est complémentaire de la pluralité de cannelures, de sorte que lorsque la première bobine interne est reçue au moins partiellement à l'intérieur de l'évidement de la première bobine externe, la pluralité de cannelures viennent en prise avec la forme complémentaire de l'évidement de la première bobine externe pour empêcher la rotation de la première bobine interne par rapport à la première bobine externe autour de l'axe longitudinal de l'arbre de bobine ; et
dans lequel le premier câble de direction est fixé à la première bobine externe et configuré pour être enroulé autour du premier ensemble de bobine lors de la rotation de la première bobine externe autour de l'axe longitudinal.

2. Système de cathéter (100) selon la revendication 1, dans lequel la première bobine interne (1300) est configurée pour être reçue à l'intérieur de la première bobine externe (1200) dans l'une quelconque d'une pluralité de positions de rotation relatives uniques, de sorte que le premier câble de direction (1400) est configuré pour avoir une première quantité de prétension dans une première de la pluralité de positions de rotation relatives uniques et une seconde quantité de prétension dans une seconde de la pluralité de positions de rotation relatives uniques, la première quantité de prétension étant différente de la seconde quantité de prétension.

3. Système de cathéter (100) selon la revendication 1 ou 2, dans lequel l'ensemble de direction (1000) comprend une base (1010), l'arbre de bobine (1100) a une partie centrale (1130) reçue à l'intérieur de la base, et le premier ensemble de bobine est positionné sur une première surface de la base.

4. Système de cathéter (100) selon la revendication 3, dans lequel l'ensemble de direction (1000) comprend un second ensemble de bobine positionné sur une seconde surface de la base (1010) opposée à la première surface de la base.

5. Système de cathéter (100) selon la revendication 4, dans lequel le second ensemble de bobine comprend une seconde bobine externe (1200), et une seconde bobine interne (1300) reçue au moins partiellement à l'intérieur d'un évidement (1220) de la seconde bobine externe.

6. Système de cathéter (100) selon la revendication 5, dans lequel la première bobine interne (1300) est identique à la seconde bobine interne (1300), et la première bobine externe (1200) est identique à la seconde bobine externe (1200).

7. Système de cathéter (100) selon la revendication 5, dans lequel l'arbre de bobine (1100) a une seconde partie d'extrémité (1110) reçue au moins partiellement à l'intérieur d'un évidement (1340) de la seconde bobine interne (1300), et la seconde partie d'extrémité de l'arbre de bobine et l'évidement de la seconde bobine interne sont chacun formés de sorte que la rotation de l'arbre de bobine autour de l'axe longitudinal de celui-ci transmette un couple à la seconde bobine interne pour provoquer la rotation de la seconde bobine interne autour de l'axe longitudinal de l'arbre de bobine.

8. Système de cathéter (100) selon la revendication 7, dans lequel la seconde bobine interne (1300) comprend une section cannelée (1330) formant une pluralité de cannelures, et l'évidement (1220) de la seconde bobine externe (1200) a une forme qui est complémentaire de la pluralité de cannelures de la section cannelée de la seconde bobine interne, de sorte que lorsque la seconde bobine interne est reçue au moins partiellement à l'intérieur de l'évidement de la seconde bobine externe, la pluralité de cannelures de la section cannelée de la seconde bobine interne viennent en prise avec la forme complémentaire de l'évidement de la seconde bobine externe pour empêcher la rotation de la seconde bobine interne par rapport à la seconde bobine externe autour de l'axe longitudinal de l'arbre de bobine.

9. Système de cathéter (100) selon la revendication 8, comprenant en outre :
une seconde lumière (412a, 412b) formée à l'intérieur de la paroi du cathéter de direction (410a, 410b) ; et
un second câble de direction (1400) s'étendant à travers la seconde lumière le long d'une longueur de la paroi du cathéter de direction vers la partie d'extrémité distale du cathéter de direction, le second câble de direction étant couplé à la partie d'extrémité distale du cathéter de direction,
dans lequel le second câble de direction est fixé à la seconde bobine externe (1200) et configuré pour être enroulé autour du second ensemble de bobine lors de la rotation de la seconde bobine externe autour de l'axe longitudinal.

10. Système de cathéter (100) selon la revendication 9, dans lequel le premier câble de direction (1400) est fixé à la première bobine externe (1200) et le second câble de direction (1400) est fixé à la seconde bobine externe (1200) de sorte que, lorsque l'arbre de bobine (1100) est mis en rotation autour de l'axe longitudinal pour provoquer la rotation simultanée de la première bobine externe et de la seconde bobine externe, le premier câble de direction s'enroule autour du premier ensemble de bobine tandis que le second câble de direction se déroule du second ensemble de bobine.

11. Système de cathéter (100) selon la revendication 10, dans lequel la première lumière (412a, 412b) est positionnée sur un côté généralement opposé du cathéter de direction (410a, 410b) par rapport à la seconde lumière (412a, 412b).

12. Système de cathéter (100) selon la revendication 11, dans lequel la tension du premier câble de direction (1400) est configurée pour dévier la partie d'extrémité distale du cathéter de direction (410a, 410b) dans une première direction de direction, et la tension du second câble de direction (1400) est configurée pour dévier la partie d'extrémité distale du cathéter de direction dans une seconde direction de direction, la première direction de direction étant généralement opposée à la seconde direction de direction.

13. Système de cathéter (100) selon la revendication 10, dans lequel la première lumière (412a, 412b) est positionnée diamétralement opposée à la seconde lumière (412a, 412b).

14. Système de cathéter (100) selon la revendication 13, dans lequel la tension du premier câble de direction (1400) est configurée pour dévier la partie d'extrémité distale du cathéter de direction (410a, 410b) dans une première direction de direction, et la tension du second câble de direction (1400) est configurée pour dévier la partie d'extrémité distale du cathéter de direction dans une seconde direction de direction, la première direction de direction et la seconde direction de direction étant positionnées dans un même plan mais dans des directions opposées le long du même plan.

15. Système de cathéter (100) selon la revendication 10, comprenant en outre un engrenage d'entraînement (1028), l'engrenage d'entraînement ayant une ouverture centrale, la seconde partie d'extrémité (1110) de l'arbre de bobine (1130) étant reçue à l'intérieur de l'ouverture centrale, dans lequel la seconde partie d'extrémité de l'arbre de bobine et l'ouverture centrale de l'engrenage d'entraînement sont chacun formés de sorte que la rotation de l'engrenage d'entraînement autour de l'axe longitudinal de l'arbre de bobine transmette un couple à l'arbre de bobine pour provoquer la rotation de l'arbre de bobine autour de l'axe longitudinal de l'arbre de bobine.
